# EUROPEAN PATENT SPECIFICATION

(11) **EP 3 019 626 B1**
(45) Date of publication and mention of the grant of the patent: **17.10.2018**
(21) Application number: 13736883.3
(22) Date of filing: 10.07.2013
(51) Int. Cl.: C12Q 1/48, C12Q 1/68, G01N 33/574, G06F 19/00

(54) **MEDICAL ANALYSIS SYSTEM FOR ASSESSING THE RISK FOR COLORECTAL CANCER**
MEDIZINISCHES ANALYSESYSTEM ZUR FESTSTELLUNG DES RISIKOS FÜR COLOREKTALKARZINOM
SYSTÈME MÉDICALE POUR L'ÉVALUATION D'UN RISQUE DE CANCER COLORECTAL

(43) Date of publication of application: 18.05.2016
(73) Proprietor: Gotthardt, Daniel, 69117 Heidelberg (DE)
(72) Inventor: WANNHOFF, Andreas, 74074 Heilbronn (DE); GOTTHARDT, Daniel, Prof., Dr., 69117 Heidelberg (DE)
(74) Representative: Richardt Patentanwälte PartG mbB
(86) International application number: PCT/EP2013/064523
(87) International publication number: WO 2015/003744

(56) References cited:
- EP-A2- 2 365 456
- WO-A2-2006/017859
- NARIMATSU ET AL: "Lewis and secretor gene dosages affect CA19-9 and DU-PAN-2 serum levels in normal individuals and colorectal cancer patients.", CANCER RESEARCH, vol. 58, no. 3, 1 February 1998 (1998-02-01), pages 512-518, XP055082070, ISSN: 0008-5472
- VESTERGAARD ET AL: "Reference values and biological variation for tumor marker CA 19-9 in serum for different Lewis and secretor genotypes and evaluation of secretor and Lewis genotyping in a Caucasian population.", CLINICAL CHEMISTRY, vol. 45, no. 1, 1 January 1999 (1999-01-01), pages 54-61, XP055082068, ISSN: 0009-9147
- ORNTOFT ET AL.: "Influence of Lewis alpha 1-3/4-L-Fucosyltransferase (FUT3) Gene Mutations on Enzyme Activity, Erythrocyte Phenotyping, and Circulating Tumor Marker Sialyl-Lewis a Levels", JOURNAL OF BIOLOGICAL CHEMISTRY, vol. 271, no. 50, 13 December 1996 (1996-12-13), pages 32260-32268, XP55081915, ISSN: 0021-9258, DOI: 10.1074/jbc.271.50.32260
- GEBAUER G ET AL: "CARCINOEMBRYONIC ANTIGEN AND CA19-9: IMPLICATIONS OF QUANTITATIVE MARKER MEASUREMENT IN TISSUES FOR PROGNOSIS OF COLORECTAL CANCER", CANCER DETECTION AND PREVENTION, ELSEVIER SCIENCE, vol. 25, no. 4, 1 January 2001 (2001-01-01), pages 344-351, XP009059690, ISSN: 0361-090X

## Description

The invention relates to a medical analysis system and a method for performing a medical analysis by a medical analysis system.

Medical analysis systems provide medical doctors and medical stuff valuable information supporting them in making medical decisions. For example, for diagnosing colorectal cancer, well known screening tests via colonoscopy are employed using a CCD camera or a fiber optic camera on a flexible tube. Even though, such an apparatus may at the same time grant the opportunity for biopsy or removal of suspected colorectal cancer lesions, this is a rather awkward procedure for diagnosing cancer.

Colorectal cancer is a cancer from uncontrolled cell growth in the colon or rectum or in the appendix. Screening is an effective means at decreasing the chance of dying from colorectal cancer and is recommended starting at the age of 50. However, screening sensitivity should be high in order to ensure acceptance of the screening in the society. This requires minimizing the number of false positive colorectal cancer results. Further, in order to increase the chance of detecting colorectal cancer, the respective screening should be made easily available at many medical practices. This requires the availability of a medical instrument for supporting the analysis of colorectal cancer indicating data which is simple and easy to handle by a doctor.

"Lewis and Secretor Gene Dosages Affect CA 19-9 and DU-PAN-2 serum levels in normal individuals and colorectal cancer patients", Cancer Research, vol. 58, no. 3, 1. Feb. 1998, pages 512-518 discusses the effect of doses of the secretor and Lewis genes on the serum levels of CA 19-9 and DU-PAN-2. It was demonstrated that the secretor gene dosage negatively affected both the CA 19-9 and DU-PAN-2 values, whereas the Lewis gene dosage positively affected the CA19-9 value and negatively affected the DU-PAN-2 value. The authors recommended revised Lewis and secretor genotype-dependent positive/negative cutoff values for CA19-9 and DU-PAN-2 to be applied for more accurate cancer diagnoses.

Clinical Chemistry, 19990101 P.B. Hoeber - ISSN 0009-9147, Vol:45, Nr:1, Page(s):54 - 61 (Vestergaard; Hein; Meyer; Grunnet; Jörgensen; Wolf; Orntoft) relates to reference values and biological variation for tumor marker CA 19-9 in serum for different Lewis and secretor genotypes and evaluation of secretor and Lewis genotyping in a Caucasian population.

Journal of Biological Chemistry, 19961213 American Society for Biochemistry and Molecular Biology - ISSN 0021-9258, Vol:271, Nr:50, Page(s):32260 - 32268 (Vestergaard E M) discusses the influence of Lewis alpha 1-3/4-L-Fucosyltransferase (FUT3) Gene Mutations on Enzyme Activity, Erythrocyte Phenotyping, and Circulating Tumor Marker Sialyl-Lewis a Levels.

Meian He et al discusses in GUT, ISSN: 0017-5749, DOI: 10.1136/gutjnl-2012-303434 q genome wide association study of genetic loci that influence tumour biomarkers cancer antigen 19-9, carcinoembryonic antigen and α fetoprotein and their associations with cancer risk.

It is an objective of the invention to provide for a medical analysis system. The invention is described by the independent claim. Preferred embodiments are described in the dependent claims.

A medical analysis system is described comprising
- a first interface being operable for receiving an antigen level, wherein the antigen level is a CA 19-9 antigen level and/or a CEA antigen level,
- a second interface being operable for receiving an FUT2 genotype and an FUT3 genotype,
- an first assigning module being operable for assigning the FUT2 genotype and the FUT3 genotype combination to a genotype group type of a set of predefined genotype group types, said assigning resulting in an assigned genotype group type,

- a selection module comprising an association of an antigen threshold level to each genotype group type of the set of predefined genotype group types, wherein the antigen threshold level is a CA 19-9 antigen threshold level and/or a CEA antigen threshold level, wherein the selection module is operable for selecting from the association the antigen threshold level associated with the assigned genotype group type and for determining if the antigen level is above the selected antigen threshold level,
- a third interface like for example a display being operable for providing an indication for a risk on colorectal cancer in case the antigen level is above the selected antigen threshold level.

Embodiments of the invention are based on the surprising insight that by determining genotype specific antigen threshold levels, the screening sensitivity for colorectal cancer is significantly enhanced compared to 'classical approaches' which consider only tumor markers by themselves. An example for a classical approach is given by Locker et. al in ASCO 2006, Update of Recommendations for the Use of Tumor Markers in the Gastrointestinal Cancer, Journal of Clinical Oncology, Vol. 24, No. 33, November 20, 2006.

Thus, embodiments of the invention may have the advantage that a medical system can be provided which makes use of this surprising insight and which could significantly reduce the number of false positive colorectal cancer results. Via the first and second interface, the medical system receives the relevant data specifying a patient's genotype and CA 19-9 antigen level and/or a CEA antigen level. CA 19-9 (cancer antigen 19-9) is a carbohydrate tumor associated antigen that may be unregulated in colorectal cancer. CEA (carcinoembryonic antigen) is a tumor associated antigen. FUT2 and FUT3 are the abbreviations for fucosyltransferase 2 or 3, respectively, which enable molecular genotyping of the human Lewis blood group system.

By sorting certain FUT2 genotype and the FUT3 genotype combination into genotype groups, a patients genetic FUT status can be taken into account when analyzing the CA 19-9 antigen level and/or a CEA antigen level of the patient. This can be done by assigning each genotype group a specific CA 19-9 antigen threshold level and/or a CEA antigen threshold level which takes into account that for example individuals lacking FUT3 activity are unable to express the CA19-9 epitope, irrespective of FUT2 activity and irrespective of the presence of colorectal cancer. In contrast, FUT2 inactivity may cause increased CA19-9 serum levels. Thus, the above mentioned antigen threshold levels may take these particularities into account increasing the chance to correctly identify colorectal cancer and at the same time minimizing the number of false positive diagnosis.

The indication on the risk for colorectal cancer may be provided in various ways. For example only the selected antigen threshold level may be provided together with the antigen level. Additionally, respective sensitivity and specificity values may be provided. Alternatively or additionally, a message may be provided indicating 'risk for colorectal cancer'.

In accordance with the invention, the first genotype group is comprising genotypes with arbitrary FUT2 gene status and FUT3:
i. homozygous mutated T202C or G508A or T1067A
ii. compound heterozygous:
   1. C314T/T202C and T59G/G508A
   2. C314T/T202C and T59G/T1067A
   3. heterozygous for all 5 SNPs
iii. homozygous mutated for T59G and heterozygous mutated for C314T or T202C or G508A or T1067A.

Further, the second genotype group is comprising genotypes with
iv. FUT3 wildtype and
   1. FUT2 homozygous wildtype or
   2. FUT2 heterozygous mutated
v. FUT2 homozygous wildtype or heterozygous mutated and FUT3:
   1. heterozygous mutated T202C (even if homozygous for C314T) or G508A or T1067A
   2. heterozygous mutated C314T/T202C or T59G/G508A or T59G/T1067 A or T59G/T202C/T1 067 A
   3. homozygous or heterozygous mutated C314T or T59G.

Further, the third genotype group comprising genotypes with a homozygous mutated FUT2 genotype status and
vi. FUT3 wildtype status or
vii. FUT3:
   1. heterozygous mutated T202C (even if homozygous for C314T) or G508A or T1067A
   2. heterozygous mutated C314T/T202C or T59G/G508A or T59G/T1067 A or T59G/T202C/T1067 A
   3. homozygous or heterozygous mutated C314T or T59G,
wherein the FUT2 wildtype status is given for FUT2 genotypes having full FUT2 activity and the FUT3 wildtype status is given for FUT3 genotypes having full FUT3 activity, wherein the FUT2 mutant status is given for FUT2 genotypes lacking FUT2 activity and the FUT3 mutant status is given for FUT3 genotypes lacking FUT3 activity.

With this set of predefined groups the operating accuracy of the above described system is optimized. The complexity of the assigning module and the selection module is retained to a manageable size thus enabling an operator of the medical analysis system to specify or tune the individual antigen threshold levels in accordance with actual insights on research on colorectal cancer.

In accordance with the invention, the antigen threshold level is increasing from the first genotype group to the second genotype group to the third genotype group. Further, for example the antigen threshold level of the first genotype group is at least a factor of 10 less than the threshold level of the second genotype group.

In accordance with an embodiment, the antigen level is a CA 19-9 antigen level and a CEA antigen level, wherein the system further comprises a second assigning module for assigning the CA 19-9 antigen level and a CEA antigen level combination to an antigen group type of a set of predefined antigen group types, said assigning resulting in an assigned antigen group type, wherein the association of the antigen threshold level to each genotype group type is further specific for each of the antigen group types, wherein the selection module is operable for selecting from the association the antigen threshold level associated with the assigned genotype group type that is specific to the assigned antigen group type.

This means that the antigen level comprises two information at the same time, namely on the CA 19-9 antigen level and a CEA antigen level. This information may depend on each other and in combination be very specific to colorectal cancer with respect to a certain genotype. Thus, this may have the advantage that a colorectal cancer specific operation of the medical analysis system can be provided at high reliability.

For example, the association of the antigen threshold level to each genotype group type is specified for each of the antigen group types via a multidimensional table, said table comprising for all combinations of the antigen group types and the genotype group types the respective antigen threshold levels. Thus, by a lookup in said table, the respective antigen threshold levels may be obtained.

In an alternative embodiment, the association of the antigen threshold level to each genotype group type is specified for each of the antigen group types via a weighting factor specified in a multidimensional table, said table comprising for all combinations of the antigen group types and the genotype group types the respective weighting factor, wherein for determining if the antigen level is above the selected antigen threshold level the selection module is operable to weight the selected antigen threshold level with the weighting factor being associated in the multidimensional table with the assigned antigen group type and the assigned genotype group type.

In accordance with an embodiment of the invention, the system further comprises a first analyzer for receiving a first blood sample, wherein the first analyzer is operable to measure the antigen level in the first blood sample and to provide the measured antigen level to the first interface. Thus, the medical analysis system may directly support the operator, for example a medical doctor, by providing a blood sample chamber for receiving a patient's blood sample. The analyzer may then directly measure the antigen level which reduces the time until receiving the indication on the risk for colorectal cancer. It has to be noted that the analyzer is not considered to be a medical diagnosing tool but a tool for supporting an operator's decision if colorectal cancer may be diagnosed or not.

In accordance with an embodiment of the invention, the system further comprises a second analyzer for receiving a second blood sample, wherein the second analyzer is operable to determine the FUT2 genotype and the FUT3 genotype and to provide the measured genotypes to the second interface.

For example, the first analyzer and the second analyzer are integrated together into a single unit. Thus, a compact medical instrument can be provided which permits an operator in an easy and quick manner to perform analysis of a patient's blood samples. This may speed up the process of a cancer diagnoses by the operator or medical doctor. Nevertheless, the analysis system may support to ensure that the number of false positive diagnoses is minimized on side of the operator.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

A 'hardware interface' or an 'interface' as used herein encompasses a interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

As will be appreciated by one skilled in the art, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magnetooptical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

The term 'analyzer' refers to a device being operable to execute one or multiple analyses on biological samples such as blood, urine, saliva, or other sample types. An analyzer is operable to determine via various chemical, biological, physical, optical or other technical procedures a parameter of the sample or a component thereof, the parameter in the following being referred to as 'measurement value'. An analyzer is operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectroscopy of proteins or metabolites and physical or chemical parameters of various type. Further, the invention may be varied to substitute colorectal cancer by pancreatic cancer.

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

In the following preferred embodiments of the invention are described in greater detail in which:
- Fig. 1: shows a block diagram of a medical analysis system,
- Fig. 2: shows a decision table of an assigning module,
- Fig. 3: shows a table of a selection module with antigen threshold levels,
- Fig. 4: shows a schematic of a medical analysis system.

In the following, similar elements are denoted by the same reference numerals.

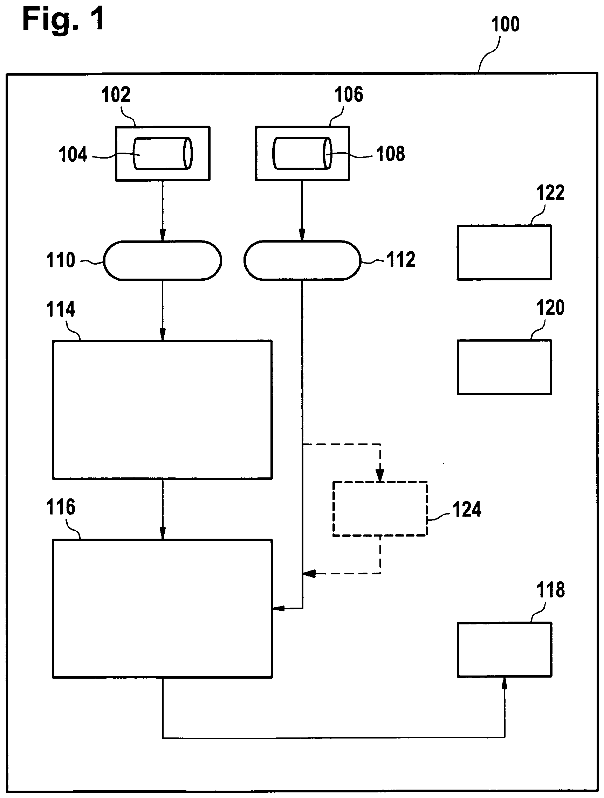

Fig. 1 shows a block diagram of a medical analysis system 100. The medical analysis system 100 comprises a first analyzer 106 adapted for receiving a blood sample 108. Further, the medical analysis system 100 comprises a second analyzer 102 adapted for receiving a blood sample 104. The second analyzer 102 is operable for determining an FUT2 genotype and an FUT3 genotype from the blood sample 104. The first analyzer 106 is operable for measuring an antigen level from the blood sample 108, like for example a CA 19-9 antigen level and/or a CEA antigen level.

The antigen level is an indicator for the presence of cancer. Low values of the antigen level suggest that a patient is cancer-free, whereas high values of the antigen level suggest that a patient has cancer.

However, based on a surprising insight it is suggested to consider a patient's genotype when trying to evaluate antigen levels for a given patient. In other words, it is suggested that antigen levels, for example CA19-9 values depend on the genotypes FUT2 and FUT3. Thus, categorizing patients as belonging to a certain genotype or genotype group can improve the sensitivity and specificity of for CA19-9 and CEA as a screening tool for colorectal cancer. Low levels of biosynthesis may result from an absence of FUT3 activity, regardless of FUT2 activity. High biosynthesis results from an absence of FUT2 activity, but no loss of FUT3 activity. Finally, intermediate biosynthesis results from all other combinations of FUT2/3. Thus, differentiation between only these three groups may improve the clinical practicability of gene-based cut-off values.

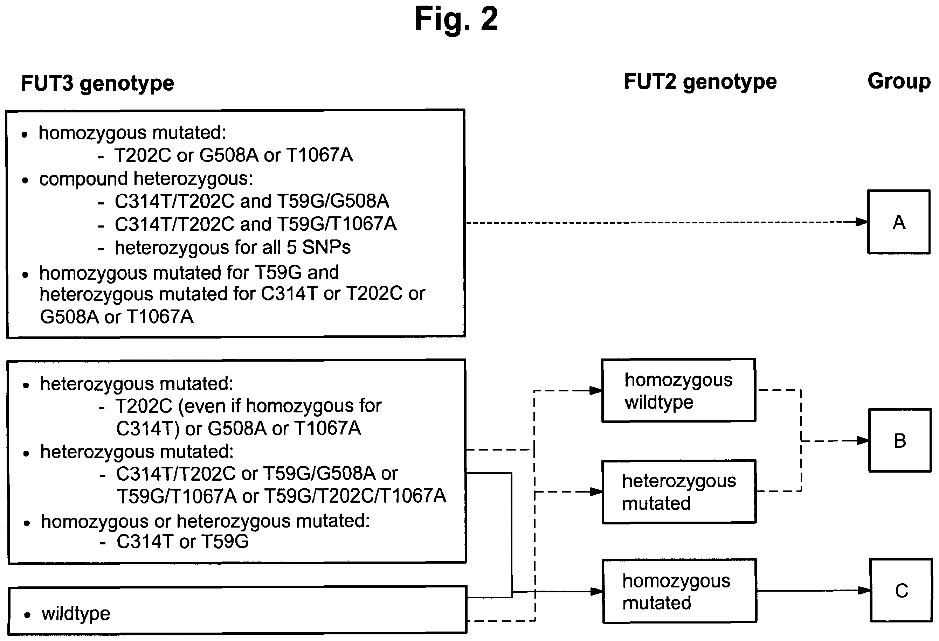

The patient's genotype combination determined via the second analyzer 102 and the antigen levels determined via the first analyzer 102 are received by the interface 110 and the interface 112, respectively. A first assigning module 114 is operable for assigning the received FUT2 genotype and the FUT3 genotype combination to a genotype group type of a set of predefined genotype group types. An example of a respective decision table of the assigning module 114 is shown in Fig. 2:
A total of three different genotype groups are predefined which can be summarized as follows:
Group A is a first genotype group comprising genotypes with a homozygous FUT3 mutation and arbitrary FUT2 gene status. Group B is a second genotype group comprising genotypes with a wildtype or heterozygous status for all allelic variants of FUT2 and FUT3. Group C is a third genotype group comprising genotypes with a homozygous FUT2 mutation and arbitrary FUT3 gene status.

The assigning module 114 is using this decision table to assign the genotypes received via the interface 110 to group A, B or C.

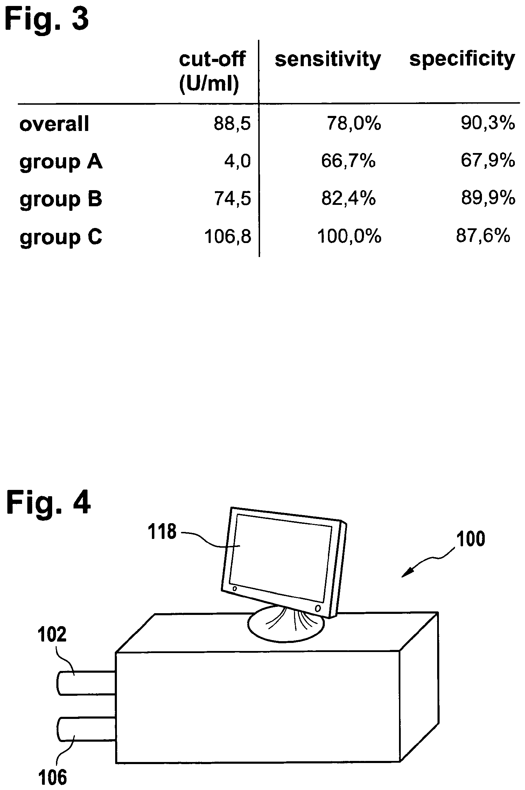

The result is then provided to a selection module 116 which comprises or makes use of a table with antigen threshold levels assigned to each group A, B or C. Such a table is exemplary illustrated in Fig. 3 for CA 19-9 antigen levels (U/ml cut-off). As can be seen, for each group, an individual antigen threshold level is available. The selection module 116 is operable to select from the table of Fig. 3 the antigen threshold level associated with the assigned genotype group type and to determine if the antigen level is above the selected antigen threshold level.

For example, if a patient belongs to group B and the measured CA 19-9 antigen level is 50 U/ml, this is an indication that the risk of colorectal cancer is low. The reason is that naturally people of group B carry an elevated level of the CA 19-9 antigen in their blood.

Contrary, in case the same patient would belong to group A and the measured CA 19-9 antigen level is 50 U/ml, this is an indication that the risk of colorectal cancer is rather high. The reason is that naturally people of group A carry a rather low level of the CA 19-9 antigen in their blood. In the latter case, the selection module 116 determines that the antigen level is above the respective threshold indicated in Fig. 3. As a consequence, the enhanced risk for colorectal cancer is indicated via the interface 118 to an operator of the system 100. The interface 118 may for example be a computer screen.

The medical analysis system 100 may further comprise a processor 120 and a memory 122. Parts or all of the above described modules may be embodied as operating instructions in software and thus stored in the memory 122 to be executed by the processor 120.

As can be seen in Fig. 3, the table assigning the antigen threshold levels to the individual genotype groups further comprises indications on sensitivity and specificity of a certain risk indication. In case an indication on a risk for colorectal cancer is provided via the third interface, the associated sensitivity and specificity of a certain risk indication may also be displayed or provided. This may support the operator of the system 100 to evaluate an indicated risk for colorectal cancer.

Not shown in Figures 2 and 3 is the possibility to use combinations of measured CA 19-9 antigen and CEA antigen levels to more specifically perform the risk evaluation on colorectal cancer. This may be done by an optional second assigning module 124 which may use a special table to perform a grouping with respect to the measured CA 19-9 antigen and CEA antigen levels. Again, multiple groups may be defined based on for example the ratio between CA 19-9 antigen and CEA antigen levels. A certain ratio range may be more specific to colorectal cancer, whereas another ratio range may be more specific for a different type of cancer like pancreatic cancer.

The antigen group determined by the second assigning module 124 may be used by the selection module 116 for selecting the antigen threshold level associated with the genotype group type determined via the first assigning module 114 and that is specific to the antigen group type determined via the second assigning module 124. For example, the selection module 116 may access a multidimensional table which assigns threshold values depending on genotype group type and antigen group type. Alternatively the selection module 116 may retrieve a weighting factor associated with a determined antigen group type from a respective table and may weigh the threshold values shown in Fig. 3 with said factor.

Figure 4 illustrates a schematic of the medical analysis system 100. Here, the first analyzer 106 and the second analyzer 102 are integrated together into a single unit. Thus, with a single medical system which in the example of fig. 4 is a single device, an easy manageable medical instrument can be provided for supporting the analysis of colorectal cancer which is simple and easy to handle by a medical doctor.

### Reference Numerals

- 100: medical system
- 102: analyzer
- 106: blood sample
- 106: analyzer
- 108: blood sample
- 110: interface
- 112: interface
- 114: assigning module
- 116: selection module
- 118: interface
- 120: processor
- 122: memory
- 124: assigning module

## Claims

1. A medical analysis system (100) comprising
- a first interface (112) being operable for receiving an antigen level, wherein the antigen level is a CA 19-9 antigen level and/or a CEA antigen level,
- a second interface (110) being operable for receiving an FUT2 genotype and an FUT3 genotype,
- a first assigning module (114) being operable for assigning the FUT2 genotype and the FUT3 genotype combination to a genotype group type of a set of predefined genotype group types, said assigning resulting in an assigned genotype group type,
- a selection module (116) comprising an association of an antigen threshold level to each genotype group type of the set of predefined genotype group types, wherein the antigen threshold level is a CA 19-9 antigen threshold level and/or a CEA antigen threshold level, wherein the selection module (116) is operable for selecting from the association the antigen threshold level associated with the assigned genotype group type and for determining if the antigen level is above the selected antigen threshold level,
- a third interface (118) being operable for providing an indication on a risk for colorectal cancer in case the antigen level is above the selected antigen threshold level,
wherein the antigen threshold level is increasing from the first genotype group to the second genotype group to the third genotype group,
- the first genotype group comprising genotypes with arbitrary FUT2 gene status and FUT3:
i. homozygous mutated T202C or G508A or T1067A
ii. compound heterozygous:
1. C314T/T202C and T59G/G508A
2. C314T/T202C and T59G/T1067A
3. heterozygous for all 5 SNPs
iii. homozygous mutated for T59G and heterozygous mutated for C314T or T202C or G508A or T1067A
- the second genotype group comprising genotypes with
i. FUT3 wildtype and
1. FUT2 homozygous wildtype or
2. FUT2 heterozygous mutated
ii. FUT2 homozygous wildtype or heterozygous mutated and FUT3:
1. heterozygous mutated T202C (even if homozygous for C314T) or G508A or T1067A
2. heterozygous mutated C314T/T202C or T59G/G508A or T59G/T1067 A or T59G/T202C/T1067 A
3. homozygous or heterozygous mutated C314T or T59G
- the third genotype group comprising genotypes with a homozygous mutated FUT2 genotype status and
i. FUT3 wildtype status or
ii. FUT3:
1. heterozygous mutated T202C (even if homozygous for C314T) or G508A or T1067A
2. heterozygous mutated C314T/T202C or T59G/G508A or T59G/T1067 A or T59G/T202C/T1067 A
3. homozygous or heterozygous mutated C314T or T59G,
wherein the FUT2 wildtype status is given for FUT2 genotypes having full FUT2 activity and the FUT3 wildtype status is given for FUT3 genotypes having full FUT3 activity, wherein the FUT2 mutant status is given for FUT2 genotypes lacking FUT2 activity and the FUT3 mutant status is given for FUT3 genotypes lacking FUT3 activity.

2. The system (100) of claim 1, wherein the antigen threshold level of the first genotype group is at least a factor of 10 less than the threshold level of the second genotype group.

3. The system (100) of any of the previous claims, wherein the antigen level is a CA 19-9 antigen level and a CEA antigen level, wherein the system (100) further comprises a second assigning module (124) for assigning the CA 19-9 antigen level and a CEA antigen level combination to an antigen group type of a set of predefined antigen group types, said assigning resulting in an assigned antigen group type, wherein the association of the antigen threshold level to each genotype group type is further specific for each of the antigen group types, wherein the selection module (116) is operable for selecting from the association the antigen threshold level associated with the assigned genotype group type that is specific to the assigned antigen group type.

4. The system (100) of claim 1, wherein the association of the antigen threshold level to each genotype group type is specified for each of the antigen group types via a multidimensional table, said table comprising for all combinations of the antigen group types and the genotype group types the respective antigen threshold levels.

5. The system (100) of claim 1, wherein the association of the antigen threshold level to each genotype group type is specified for each of the antigen group types via a weighting factor specified in a multidimensional table, said table comprising for all combinations of the antigen group types and the genotype group types the respective weighting factor, wherein for determining if the antigen level is above the selected antigen threshold level the selection module (116) is operable to weight the selected antigen threshold level with the weighting factor being associated in the multidimensional table with the assigned antigen group type and the assigned genotype group type.

6. The system (100) of any of the previous claims, further comprising a first analyzer (106) for receiving a first blood sample (108), wherein the first analyzer (106) is operable to measure the antigen level in the first blood sample (108) and to provide the measured antigen level to the first interface (112).

7. The system (100) of any of the previous claims, further comprising a second analyzer (102) for receiving a second blood sample (104), wherein the second analyzer (102) is operable to determine the FUT2 genotype and the FUT3 genotype and to provide the measured genotypes to the second interface (110).

8. The system (100) of claim 6, wherein the first analyzer (106) and the second analyzer (102) are integrated together into a single unit.

9. A method for performing a medical analysis by a medical analysis system (100), the method comprising:
- receiving by a first interface (112) of the medical analysis system (100) an antigen level, wherein the antigen level is a CA 19-9 antigen level and/or a CEA antigen level,
- receiving by a second interface (110) of the medical analysis system (100) an FUT2 genotype and an FUT3 genotype,
- assigning by a first assigning module (114) of the medical analysis system (100) the FUT2 genotype and the FUT3 genotype combination to a genotype group type of a set of predefined genotype group types, said assigning resulting in an assigned genotype group type,
- determining by a selection module (116) of the medical analysis system (100) from the association the antigen threshold level associated with the assigned genotype group type and for determining if the antigen level is above the determined antigen threshold level, wherein the selection module (116) comprises an association of an antigen threshold level to each genotype group type of the set of predefined genotype group types, wherein the antigen threshold level is a CA 19-9 antigen threshold level and/or a CEA antigen threshold level,
- providing a control signal via a third interface (118) of the medical analysis system (100) in case the antigen level is above the determined antigen threshold level,
wherein the antigen threshold level is increasing from the first genotype group to the second genotype group to the third genotype group, wherein
- the first genotype group comprises genotypes with arbitrary FUT2 gene status and FUT3:
i. homozygous mutated T202C or G508A or T1067A
ii. compound heterozygous:
1. C314T/T202C and T59G/G508A
2. C314T/T202C and T59G/T1067A
3. heterozygous for all 5 SNPs
iii. homozygous mutated for T59G and heterozygous mutated for C314T or T202C or G508A or T1067A
- the second genotype group comprises genotypes with
i. FUT3 wildtype and
1. FUT2 homozygous wildtype or
2. FUT2 heterozygous mutated
ii. FUT2 homozygous wildtype or heterozygous mutated and FUT3:
1. heterozygous mutated T202C (even if homozygous for C314T) or G508A or T1067A
2. heterozygous mutated C314T/T202C or T59G/G508A or T59G/T1067 A or T59G/T202C/T1067 A
3. homozygous or heterozygous mutated C314T or T59G
- the third genotype group comprises genotypes with a homozygous mutated FUT2 genotype status and
i. FUT3 wildtype status or
ii. FUT3:
1. heterozygous mutated T202C (even if homozygous for C314T) or G508A or T1067A
2. heterozygous mutated C314T/T202C or T59G/G508A or T59G/T1067 A or T59G/T202C/T1067 A
3. homozygous or heterozygous mutated C314T or T59G,
wherein the FUT2 wildtype status is given for FUT2 genotypes having full FUT2 activity and the FUT3 wildtype status is given for FUT3 genotypes having full FUT3 activity, wherein the FUT2 mutant status is given for FUT2 genotypes lacking FUT2 activity and the FUT3 mutant status is given for FUT3 genotypes lacking FUT3 activity.

## Patentansprüche

1. Ein medizinisches Analysesystem (100), umfassend:
- eine erste Grenzfläche (112), die für die Aufnahme eines Antigenspiegels betriebsbereit ist, wobei der Antigenspiegel ein CA 19-9-Antigenspiegel und/oder ein CEA-Antigenspiegel ist,
- eine zweite Grenzfläche (110), die für die Aufnahme eines FUT2-Genotyps und eines FUT3-Genotyps betriebsbereit ist,
- ein erstes Zuordnungsmodul (114), das für die Zuordnung der Kombination aus dem FUT2-Genotyp und dem FUT3-Genotyp zu einem Genotypgruppentyp eines Sets zuvor definierter Genotypgruppentypen betriebsbereit ist, wobei die Zuordnung in einem zugeordneten Genotypgruppentyp resultiert,
- ein Auswahlmodul (116), das eine Verknüpfung eines Antigenschwellenspiegels mit jedem Genotypgruppentyp des Sets zuvor definierter Genotypgruppentypen umfasst, wobei der Antigenschwellenspiegel ein CA 19-9-Antigenschwellenspiegel und/oder ein CEA-Antigenschwellenspiegel ist, wobei das Auswahlmodul (116) betriebsbereit ist für die Auswahl des mit dem zugeordneten Genotypgruppentyp assoziierten Antigenschwellenspiegels aus der Verknüpfung und für die Bestimmung, ob der Antigenspiegel über dem ausgewählten Antigenschwellenspiegel liegt,
- eine dritte Grenzfläche (118), die für die Bereitstellung einer Angabe zu einem Risiko für Kolorektalkrebs betriebsbereit ist, falls der Antigenspiegel über dem ausgewählten Antigenschwellenspiegel liegt,
wobei der Antigenschwellenspiegel von der ersten Genotypgruppe zur zweiten Genotypgruppe zur dritten Genotypgruppe ansteigt,
- die erste Genotypgruppe, umfassend: Genotypen mit beliebigem FUT2-Genstatus und FUT3:
i. homozygot mutiert T202C oder G508A oder T1067A
ii. zusammengesetzt heterozygot:
1. C314T/T202C und T59G/G508A
2. C314T/T202C und T59G/T1067A
3. heterozygot für alle 5 SNPs
iii. homozygot mutiert für T59G und heterozygot mutiert für C314T oder T202C oder G508A oder T1067A
- die zweite Genotypgruppe, umfassend: Genotypen mit
i. FUT3-Wildtyp und
1. FUT2 homozygot Wildtyp oder
2. FUT2 heterozygot mutiert
ii. FUT2 homozygot Wildtyp oder heterozygot mutiert und FUT3:
1. heterozygot mutiert T202C (selbst wenn für C314T homozygot) oder G508A oder T1067A
2. heterozygot mutiert C314T/T202C oder T59G/G508A oder T59G/T1067A oder T59G/T202C/T1067A
3. homozygot oder heterozygot mutiert C314T oder T59G
- die dritte Genotypgruppe, umfassend: Genotypen mit homozygotem mutiertem FUT2-Genotypstatus und
i. FUT3-Wildtypstatus oder
ii. FUT3:
1. heterozygot mutiert T202C (selbst wenn für C314T homozygot) oder G508A oder T1067A
2. heterozygot mutiert C314T/T202C oder T59G/G508A oder T59G/T1067A oder T59G/T202C/T1067A
3. homozygot oder heterozygot mutiert C314T oder T59G,
wobei der FUT2-Wildtypstatus für FUT2-Genotypen mit vollständiger FUT2-Aktivität gegeben ist und der FUT3-Wildtypstatus für FUT3-Genotypen mit vollständiger FUT3-Aktivität gegeben ist, wobei der FUT2-Mutantenstatus für FUT2-Genotypen ohne FUT2-Aktivität gegeben ist und der FUT3-Mutantenstatus für FUT3-Genotypen ohne FUT3-Aktivität gegeben ist.

2. Medizinisches Analysesystem (100) gemäß Anspruch 1, wobei der Antigenschwellenspiegel der ersten Genotypgruppe mindestens um einen Faktor 10 geringer ist als der Schwellenspiegel der zweiten Genotypgruppe.

3. System (100) gemäß einem der vorhergehenden Ansprüche, wobei der Antigenspiegel ein CA 19-9-Antigenspiegel und ein CEA-Antigenspiegel ist, wobei das System (100) ferner ein zweites Zuordnungsmodul (124) für die Zuordnung der Kombination aus dem CA 19-9-Antigenspiegel und dem CEA-Antigenspiegel zu einem Antigengruppentyp eines Sets zuvor definierter Antigengruppentypen umfasst, wobei die Zuordnung in einem zugeordneten Antigengruppentyp resultiert, wobei die Verknüpfung des Antigenschwellenspiegels mit jedem Genotypgruppentyp ferner für jeden der Antigengruppentypen spezifisch ist, wobei das Auswahlmodul (116) betriebsbereit ist für die Auswahl des Antigenschwellenspiegels, der mit dem für den zugeordneten Antigengruppentyp spezifischen zugeordneten Genotypgruppentyp assoziiert ist, aus der Verknüpfung.

4. System (100) gemäß Anspruch 1, wobei die Verknüpfung des Antigenschwellenspiegels mit jedem Genotypgruppentyp anhand einer multidimensionalen Tabelle für jeden der Antigengruppentypen spezifiziert wird, wobei die Tabelle für alle Kombinationen aus den Antigengruppentypen und den Genotypgruppentypen den entsprechenden Antigenschwellenspiegel umfasst.

5. System (100) gemäß Anspruch 1, wobei die Verknüpfung des Antigenschwellenspiegels mit jedem Genotypgruppentyp anhand eines in einer multidimensionalen Tabelle spezifizierten Gewichtungsfaktors für jeden der Antigengruppentypen spezifiziert wird, wobei die Tabelle für alle Kombinationen aus den Antigengruppentypen und den Genotypgruppentypen den entsprechenden Gewichtungsfaktor umfasst, wobei zur Bestimmung dessen, ob der Antigenspiegel über dem ausgewählten Antigenschwellenspiegel liegt, das Auswahlmodul (116) betriebsbereit ist, den ausgewählten Antigenschwellenspiegel zu gewichten, wobei der Gewichtungsfaktor in der multidimensionalen Tabelle mit dem zugeordneten Antigengruppentyp und dem zugeordneten Genotypgruppentyp assoziiert ist.

6. System (100) gemäß einem der vorhergehenden Ansprüche, ferner umfassend ein erstes Analysegerät (106) zur Aufnahme einer ersten Blutprobe (108), wobei das erste Analysegerät (106) betriebsbereit ist, um den Antigenspiegel in der ersten Blutprobe (108) zu messen und den gemessenen Antigenspiegel der ersten Grenzfläche (112) bereitzustellen.

7. System (100) gemäß einem der vorhergehenden Ansprüche, ferner umfassend ein zweites Analysegerät (102) zur Aufnahme einer zweiten Blutprobe (104), wobei das zweite Analysegerät (102) betriebsbereit ist, um den FUT2-Genotyp und den FUT3-Genotyp zu bestimmen und die gemessenen Genotypen der zweiten Grenzfläche (110) bereitzustellen.

8. System (100) gemäß Anspruch 6, wobei das erste Analysegerät (106) und das zweite Analysegerät (102) in einer einzigen Einheit zusammen integriert sind.

9. Ein Verfahren zur Durchführung einer medizinischen Analyse anhand eines medizinischen Analysesystems (100), wobei das Verfahren Folgendes umfasst:
- Aufnehmen, durch eine erste Grenzfläche (112) des medizinischen Analysesystems (100), eines Antigenspiegels, wobei der Antigenspiegel ein CA 19-9-Antigenspiegel und/oder ein CEA-Antigenspiegel ist,
- Aufnehmen, durch eine zweite Grenzfläche (110) des medizinischen Analysesystems (100), eines FUT2-Genotyps und eines FUT3-Genotyps,
- Zuordnen, anhand eines ersten Zuordnungsmoduls (114) des medizinischen Analysesystems (100), der Kombination aus dem FUT2-Genotyp und dem FUT3-Genotyp zu einem Genotypgruppentyp eines Sets zuvor definierter Genotypgruppentypen, wobei die Zuordnung in einem zugeordneten Genotypgruppentyp resultiert,
- Bestimmen, anhand eines Auswahlmoduls (116) des medizinischen Analysesystems (100), des mit dem zugeordneten Genoptypgruppentyp assoziierten Antigenschwellenspiegels aus der Verknüpfung und Bestimmen, ob der Antigenspiegel über dem bestimmten Antigenschwellenspiegel liegt, wobei das Auswahlmodul (116) eine Verknüpfung eines Antigenschwellenspiegels mit jedem Genotypgruppentyp des Sets zuvor definierter Genotypgruppentypen umfasst, wobei der Antigenschwellenspiegel ein CA 19-9-Antigenschwellenspiegel und/oder ein CEA-Antigenschwellenspiegel ist,
- Bereitstellen eines Steuersignals durch eine dritte Grenzfläche (118) des medizinischen Analysesystems (100) für den Fall, dass der Antigenspiegel über dem bestimmten Antigenschwellenspiegel liegt,
wobei der Antigenschwellenspiegel von der ersten Genotypgruppe zur zweiten Genotypgruppe zur dritten Genotypgruppe ansteigt, wobei
- die erste Genotypgruppe Genotypen mit beliebigem FUT2-Genstatus und FUT3 umfasst:
i. homozygot mutiert T202C oder G508A oder T1067A
ii. zusammengesetzt heterozygot:
1. C314T/T202C und T59G/G508A
2. C314T/T202C und T59G/T1067A
3. heterozygot für alle 5 SNPs
iii. homozygot mutiert für T59G und heterozygot mutiert für C314T oder T202C oder G508A oder T1067A
- die zweite Genotypgruppe Genotypen mit Folgendem umfasst:
i. FUT3-Wildtyp und
1. FUT2 homozygot Wildtyp oder
2. FUT2 heterozygot mutiert
ii. FUT2 homozygot Wildtyp oder heterozygot mutiert und FUT3:
1. heterozygot mutiert T202C (selbst wenn für C314T homozygot) oder G508A oder T1067A
2. heterozygot mutiert C314T/T202C oder T59G/G508A oder T59G/T1067A oder T59G/T202C/T1067A
3. homozygot oder heterozygot mutiert C314T oder T59G
- die dritte Genotypgruppe Genotypen mit homozygotem mutiertem FUT2-Genotypstatus und Folgendem umfasst:
i. FUT3-Wildtypstatus oder
ii. FUT3:
1. heterozygot mutiert T202C (selbst wenn für C314T homozygot) oder G508A oder T1067A
2. heterozygot mutiert C314T/T202C oder T59G/G508A oder T59G/T1067A oder T59G/T202C/T1067A
3. homozygot oder heterozygot mutiert C314T oder T59G,
wobei der FUT2-Wildtypstatus für FUT2-Genotypen mit vollständiger FUT2-Aktivität gegeben ist und der FUT3-Wildtypstatus für FUT3-Genotypen mit vollständiger FUT3-Aktivität gegeben ist, wobei der FUT2-Mutantenstatus für FUT2-Genotypen ohne FUT2-Aktivität gegeben ist und der FUT3-Mutantenstatus für FUT3-Genotypen ohne FUT3-Aktivität gegeben ist.

## Revendications

1. Système d'analyse médicale (100) comprenant
- une première interface (112) ayant pour fonction de recevoir un taux d'antigène, où le taux d'antigène est un taux d'antigène CA 19-9 et/ou un taux d'antigène ACE,
- une deuxième interface (110) ayant pour fonction de recevoir un génotype FUT2 et un génotype FUT3,
- un premier module d'attribution (114) ayant pour fonction d'attribuer la combinaison du génotype FUT2 et du génotype FUT3 à un type de groupe de génotypes d'un ensemble de types de groupe de génotypes prédéfini, ladite attribution ayant pour résultat un type de groupe de génotypes attribué,
- un module de sélection (116) comprenant une association d'un taux limite d'antigène à chaque type de groupe de génotypes de l'ensemble des types de groupes de génotypes prédéfinis, où le taux limite d'antigène est un taux limite d'antigène CA 19-9 et/ou un taux limite d'antigène ACE, où le module de sélection (116) peut avoir la fonction de choisir, à partir de l'association, le taux limite d'antigène associé avec le type de groupe de génotypes attribué et pour déterminer si le taux d'antigène est supérieur au taux limite d'antigène choisi,
- une troisième interface (118) ayant pour fonction de fournir une indication d'un risque de cancer colorectal dans le cas où le taux d'antigène est supérieur au taux limite d'antigène choisi,
où le taux limite d'antigène augmente à partir du premier groupe de génotypes au deuxième groupe de génotypes jusqu'au troisième groupe de génotypes,
- le premier groupe de génotypes comprenant des génotypes avec un état de gène FUT2 arbitraire et FUT3 :
i. homozygote muté T202C, ou G508A, ou T1067A
ii. hétérozygote composé :
1. C314T/T202C et T59G/G508A
2. C314T/T202C et T59G/T1067A
3. hétérozygote pour tous les 5 polymorphismes nucléotidiques SNP iii homozygote muté pour T59G et hétérozygote muté pour C314T, ou T202C, ou G508A, ou T1067A,
- le deuxième groupe de génotypes comprenant des génotypes avec
i. un FUT3 de type sauvage et
1. homozygote FUT2 de type sauvage ou
2. hétérozygote muté FUT2
ii. un homozygote FUT2 de type sauvage ou un hétérozygote muté et FUT3 :
1. hétérozygote muté T202C (même si homozygote pour C314T), ou G508A, ou T1067A
2. hétérozygote muté C314/T202C, ou T59G/G508A, ou T59G/T1067A, ou T59G/T202C/T1067A
3. homozygote ou hétérozygote muté C314T ou T59G
- le troisième groupe de génotypes comprenant des génotypes avec un état de génotype FUT2 homozygote muté et
i. un FUT3 de type sauvage ou
ii. un FUT3 :
1. hétérozygote muté T202C (même si homozygote pour C314T), ou G508A, ou T1067A
2. hétérozygote muté C314/T202C, ou T59G/G508A, ou T59G/T1067A, ou T59G/T202C/T1067A
3. homozygote ou hétérozygote muté C314T ou T59G,
où l'état FUT2 de type sauvage est donné pour des génotypes FUT2 ayant une activité FUT2 totale et l'état FUT3 de type sauvage est donné pour des génotypes FUT3 ayant une activité FUT3 complète, où l'état mutant de FUT2 est donné pour des génotypes FUT2 manquant d'activité FUT2 et l'état muté FUT3 est donné pour des génotypes FUT3 manquant d'activité FUT3.

2. Système (100) selon la revendication 1, dans lequel le taux limite d'antigène du premier groupe de génotypes est inférieur au moins un facteur 10 au taux limite du second groupe de génotypes.

3. Système (100) selon l'une quelconque des revendications précédentes, dans lequel le taux d'antigène est un taux d'antigène CA 19-9 et un taux d'antigène ACE, où le système (100) comprend en outre un second module d'attribution (124) pour attribuer la combinaison du taux d'antigène CA 19-9 et du taux d'antigène ACE à un type de groupe d'antigènes d'un ensemble de types de groupes d'antigènes prédéfini, ladite attribution ayant pour résultat un type de groupe d'antigènes attribué, où l'association du taux limite d'antigène à chaque type de groupes de génotypes est en outre spécifique pour chacun des types de groupes d'antigènes, où le module de sélection (116) peut avoir pour fonction de choisir parmi l'association le taux limite d'antigène associé avec le type de groupe de génotypes attribué qui est spécifique au type de groupe d'antigènes attribué.

4. Système (100) selon la revendication 1, dans lequel l'association du taux limite d'antigène à chaque type de groupe de génotypes est spécifiée pour chacun des types de groupes d'antigènes par le biais d'un tableau multidimensionnel, ledit tableau comprenant toutes les combinaisons des types de groupes d'antigènes et des types de groupes de génotypes des taux limites de l'antigène respectifs.

5. Système (100) selon la revendication 1, dans lequel l'association du taux limite d'antigène à chaque type de groupe de génotypes est spécifiée pour chacun des types de groupes d'antigènes par le biais d'un facteur de pondération spécifié dans un tableau multidimensionnel, ledit tableau comprenant pour toutes les combinaisons des types de groupes d'antigènes et des types de groupes de génotypes, le facteur de pondération respectif, où, pour déterminer si le taux d'antigène est supérieur au taux limite d'antigène sélectionné, le module de sélection (116) peut avoir pour fonction de pondérer le taux limite d'antigène sélectionné avec le facteur de pondération étant associé dans le tableau multidimensionnel avec le type de groupe d'antigènes attribué et le type de groupe de génotypes attribué.

6. Système (100) selon l'une quelconque des revendications précédentes, comprenant en outre un premier analyseur (106) pour recevoir un premier échantillon de sang (108), où le premier analyseur (106) peut avoir pour fonction de mesurer le taux d'antigènes dans le premier échantillon de sang (108) et pour fournir le taux d'antigène mesuré à la première interface (112).

7. Système (100) selon l'une quelconque des revendications précédentes, comprenant en outre un second analyseur (102) pour recevoir un second échantillon de sang (104), où le second analyseur (102) peut avoir pour fonction de déterminer le génotype FUT2 et le génotype FUT3 et pour fournir les génotypes mesurés à la seconde interface (110).

8. Système (100) selon la revendication 6, dans lequel le premier analyseur (106) et le second analyseur (102) sont intégrés ensemble dans une entité unique.

9. Procédé de réalisation d'une analyse médicale par un système d'analyse médicale (100), le procédé comprenant :
- la réception, par une première interface (112) du système d'analyse médicale (100), d'un taux d'antigène, où le taux d'antigène est un taux d'antigène CA 19-9 et/ou un taux d'antigène ACE,
- la réception, par une deuxième interface (110) du système d'analyse médicale (100), d'un génotype FUT2 et d'un génotype FUT3,
- l'attribution, par un premier module d'attribution (114) du système d'analyse médicale (100), de la combinaison du génotype FUT2 et du génotype FUT3 à un type de groupe de génotypes d'un ensemble de types de groupes de génotypes prédéfini, ladite attribution ayant pour résultat un type de groupe de génotypes attribué,
- la détermination, par un module de sélection (116) du système d'analyse médicale (100), à partir de l'association, le taux limite d'antigène associé avec le type de groupe de génotypes associé et pour déterminer si le taux d'antigène est supérieur au taux limite d'antigène déterminé, où le module de sélection (116) comprend une association d'un taux limite d'antigène à chaque type de groupe de génotypes de l'ensemble de types de groupes de génotypes prédéfini, où le taux limite d'antigène est un taux limite d'antigène CA 19-9 et/ou un taux limite d'antigène ACE,
- la fourniture d'un signal de commande par le biais d'une troisième interface (118) du système d'analyse médicale (100) dans le cas où le taux d'antigène est supérieur au taux limite d'antigène déterminé,
où le taux limite d'antigène augmente du premier groupe de génotypes au deuxième groupe de génotypes jusqu'au troisième groupe de génotypes, où
- le premier groupe de génotypes comprend des génotypes avec un état de gène FUT arbitraire et FUT3 :
i. un homozygote muté T202C ou G508A, ou T1067A
ii. un hétérozygote composé :
1. C314T/T202C et T59G/G508A
2. C314T/T202C et T59G/T1067A
3. hétérozygote pour tous les 5 polymorphismes nucléotidiques SNP
iii un homozygote muté pour T59G et un hétérozygote muté pour C314T, ou T202C, ou G508A, ou T1067A
- le deuxième groupe de génotypes comprend des génotypes avec
i. un FUT3 de type sauvage et
1. homozygote FUT2 de type sauvage ou
2. hétérozygote muté FUT2
ii. un homozygote FUT2 de type sauvage ou un hétérozygote muté et FUT3 :
1. hétérozygote muté T202C (même si homozygote pour C314RT), ou G508A, ou T1067A
2. hétérozygote muté C314/T202C, ou T59G/G508A, ou T59G/T1067A, ou T59G/T202C/T1067A
3. homozygote ou hétérozygote muté C314T ou T59G
- le troisième groupe de génotypes comprend des génotypes avec un état de génotype FUT2 homozygote muté et
i. un FUT3 de type sauvage ou
ii. un FUT3 :
1. hétérozygote muté T202C (même si homozygote pour C314T), ou G508A, ou T1067A
2. hétérozygote muté C314/T202C, ou T59G/G508A, ou T59G/T1067A, ou T59G/T202C/T1067A
3. homozygote ou hétérozygote muté C314T ou T59G,
où l'état FUT2 de type sauvage est donné pour des génotypes FUT2 ayant une activité FUT2 complète et l'état FUT3 de type sauvage est donné pour des génotypes FUT3 ayant une activité FUT3 complète, où l'état mutant de FUT2 est donné pour des génotypes FUT2 manquant d'activité FUT2 et l'état muté FUT3 est donné pour des génotypes FUT3 manquant d'activité FUT3.
